# EUROPEAN PATENT APPLICATION

(11) **EP 3 845 946 A1**
(43) Date of publication of application: **07.07.2021**
(21) Application number: 19854951.1
(22) Date of filing: 30.08.2019
(51) Int. Cl.: G02B 23/24

(54) **ANTI-FOGGING SYSTEM THAT CAN BE THERMOREGULATED BY MEANS OF A MICRO CONTROLLER FOR RIGID ENDOSCOPES**

(30) Priority: 31.08.2018 ES 201830858
(71) Applicant: Noruco, S.L., 41007 Sevilla (ES)
(72) Inventor: RUIZ CORRALES, Juan Luis, 41007 Sevilla (ES)
(74) Representative: Pons
(86) International application number: PCT/ES2019/070582
(87) International publication number: WO 2020/043928

(57) **Abstract**

The end result, with the combination of an anti-fogging system that can be thermoregulated by means of a micro controller, for rigid endoscopes, which is the subject matter of the invention, is to have an apparently standard rigid endoscope (fig. 4) that can withstand continuous temperature changes during surgical intervention, imparting value to the endoscopic device and preventing general fogging. With this thermoregulatable anti-fogging system, temperature levels can be controlled, it thereby being easy to prevent fogging/condensation in any intervention where a rigid endoscope is used, such as hysteroscopy, cystoscopy, general surgery, arthroscopy, etc. Using the invention completely prevents the need for the surgeon to remove the endoscope from inside the patient in the middle of surgery. Thus, the surgeon does not become fatigued by continuous poor visibility. This reduces the duration of the intervention, optimising the process and providing the quality of vision anticipated for the endoscope in question.

## Description

### TECHNICAL SECTOR

The present invention belongs to the field of medical-surgical equipment and more specifically to that of minimally invasive surgery.

The main object of the present invention is a system that can be thermoregulated by means of a micro controller, which is implemented in the manufacturing process of any rigid endoscope. Through which we can control the temperature levels of the distal end, with the object of preventing fogging/condensation in any intervention wherein a rigid endoscope is used, such as in hysteroscopy, urology, general surgery, arthroscopy, etc.

### BACKGROUND OF THE INVENTION

Endoscopic surgery is a common and safe medical technique today. It is a surgical technique which is practised through a natural orifice, a surgical incision or an injury, for the visualisation of a hollow organ or body cavity. Using the assistance of a video camera enables the medical team to see the surgical field inside the patient and perform the intervention on them. Light is transmitted through fibreglass for illumination and visualisation without transmitting harmful heat.

These techniques are called minimally invasive, since they prevent the large scalpel cuts required by open or conventional surgery and therefore enable a much faster and more comfortable post-operative period.

Endoscopic procedures for the diagnosis and treatment of pathologies have been developed until they have become common procedures in all hospitals and medical centres.

Rigid endoscopy is currently a common and safe diagnostic-surgical technique. It is a surgical technique which is practised through a natural orifice, a surgical incision or an injury, for the visualisation of a hollow organ or body cavity. Using the assistance of a video camera enables the medical team to see the surgical field inside the patient and perform the intervention on them. These techniques are known as minimally invasive since they prevent large interventions and, therefore, enable a recovery period with much fewer comorbidities and being more comfortable for the patient.

The endoscopic treatment of injuries currently encompasses a variety of techniques, such as laparoscopy, for visualising the inner contents of the abdominal cavity; arthroscopy, for joints; cystoscopy, for the inner surface of the urinary bladder and urethra; and hysteroscopy, for the inside of the uterus through the vagina and the cervix.

Since the beginning of endoscopic surgery, vision problems during surgery have been a handicap in the development of minimally invasive techniques. Maintaining a correct vision during the surgery is crucial for maintaining the efficacy and safety of the technique. These deficits in the vision can lead to increases in operating time or even the appearance of complications. Some of these vision problems have improved in parallel with the development of vision systems with greater definition, both in the optics and in the monitors. But there are some vision problems derived from the fogging of the lenses which is inherent to the surgery due to the difference in temperature between the inside of the patient and the lens (Lens Fogging, LF). Lawrentschuk et al demonstrated in an experimental study that the vision problems derived from LF during laparoscopy increased as the temperature in the lens of the optic decreased, and increased as the percentage of intraabdominal humidity increased.

The fogging of the tip of the endoscope or distal end (LF) is caused by the difference in temperature between the two faces of the glass of the tip. Air has a capacity to retain humidity in the form of vapour which has a direct relationship with the temperature. Thus, when a volume of air which retains a certain volume of water vapour cools, the retention capacity thereof will decrease and the excess humidity will condense. The film of air which is in contact with a cold surface is forced to shed the excess humidity and it is this condensation which fogs the glass of the endoscope. The following cause the fogging of the lens: the low temperature of the insufflated gas (CO2), which is used in certain surgeries such as gastric surgery; the body temperature itself of the patient; the humidity existing inside the surgical field due to the presence of bodily fluids; the interaction of surgical instruments and the endoscope; and external variables.

Multiple solutions have been proposed in order to prevent this problem. Among the options proposed to decrease LF, we can find 3 large groups:
1. Mechanisms for heating the endoscope. Different methods have been described for maintaining the end of the endoscope at a temperature which decreases fogging. The use of externally heated and humidified CO2 was proposed as an efficient theoretical method for reducing LF, but without a real clinical demonstration. Classically, the use of hot saline solution to increase the temperature of the endoscope and even the use of thermoses to keep the serum hot for a longer time have been described. Devices have also been developed which heat the tip of the optic when it is dry.
2. Anti-fogging solutions. Several products have been used to reduce the propensity for fogging by using surfactant substances in the tip of the laparoscope. Classics like Betadine or chlorhexidine or more specifically like FREDTM (Covidien, Dublin, Ireland), ResoclearTM (Resorba, Nurnberg, Germany).
3. Modifications to the equipment of the endoscope. Different modifications to the conventional endoscope have been described for decreasing LF. The Ohdaira group described the use of a layer of titanium dioxide on the glass of the optic in order to decrease the fogging of the camera, and a vibration system in the tip of the optic performed the self-cleaning. One of the main drawbacks that have interfered in the development and commercialisation thereof is the high price and the problems with the sterilisation of the endoscope.

However, these are only temporary solutions, since the moment the optic loses temperature, the lens fogs up again. These unsolved difficulties cause: loss of vision in the surgical or examined field; loss of time during the intervention caused by the continuous repetitions in the extraction and insertion of the endoscope in the body during the intervention in order to defog it and continue with the surgical process; risk of contamination of the equipment and/or the patient; increase in the fatigue of users during the surgical process, among others.

As seen so far, most publications and studies focus on methods for externally heating the tip of the endoscope, either with different solutions at different temperatures or local heating systems. Given that the most important factor for the onset of LF is the difference in temperature between the optic and the inside of the patient, a regulatable self-heating system has been developed which maintains a constant temperature of the endoscope throughout the intervention regardless of the conditions produced during the same, and thus eliminating the LF and improving the quality of the vision of the surgeon during the intervention.

Any person skilled in the art would think to work on the image in order to solve the problem of fogging, in other words, applying solutions to the image components, such as the objective lens, the lenses, the camera and the monitor. In the present invention, the image is not worked on, however, we manage to improve the quality of vision without modifying the external and internal dimensions of the product.

Unlike what is described in documents CN20538610 U and US2007149856 A1, wherein the electrical resistor is not nichrome and wherein the electrical resistor is located inside the internal tube, in the medical device for rigid endoscopy which is described in this patent application, the electrical resistor is located outside of said internal tube.

Another important difference between the device described herein and CN20538610 U is that the latter includes a single tube, as opposed to the double coaxial tube structure comprised in the device of the present invention.

Other differences of the invention with respect to documents CN20538610 U and US2007149856 A1 relate to the fact that the electrical resistor is fully adhered and insulated by means of a tape stuck to the outer surface of the internal tube, at the same time that the optical fibre is also located inside the coaxial space enveloping said electrical resistor.

Thus, in the present invention, the nichrome electrical resistor is located in the coaxial space delimited between the internal tube and the external tube; wherein the optical fibre passes through said coaxial space and is also located (which is separated by an insulator), in comparison with documents CN20538610 U and US2007149856 A1, wherein the electrical resistor (which is not made of nichrome) goes inside the internal tube, along with the lenses.

In this situation, what no one would have thought of is the strategic location of the electrical resistor, wherein it provides a controlled temperature, thus protecting the lenses and the objective lens, contrary to what happens in CN20538610 U and US2007149856 A1, which were never manufactured, wherein the invention is a component of the system of the image (the objective lens), causing unwanted temperature increases of the objective lens, and therefore, the application thereof in the market being impossible.

Moreover, it should be mentioned that the main problem of deterioration of an endoscope is the inlet of humidity and the loss of the seal. When this inlet of humidity or fluids occurs, said inlet is produced directly through the lens system. In the device of the present invention, the electrical resistor is located outside of the lens system and not in direct contact with it, thus preventing the electrical risk.

In contrast, in conventional endoscopes such as the ones described in documents CN20538610 U and US2007149856 A1, if humidity or a liquid enters the space wherein the electrical resistor and the lens system are jointly located, said electrical resistor would get wet, becoming broken or damaged, which would cause an electrical risk for the product and for the connection to the thermoregulatable controller.

To all this it should be added that the electrical resistor described here does not imply any change in the standard of the product. The same lens system with certified quality can still be used, enabling the easy repair of the lens system in the case of deterioration due to the seal, without influencing the electrical system that it has installed between the internal tube and the external tube.

It should also be noted that we have information that the endoscope apparatuses such as the ones described in CN20538610 U and US2007149856 A1 are not manufactured due to the impossibility of adaptation, such that without modifying the standards of the product and the conventional common parts, it is impossible to manufacture in this manner; among other things because there is not enough space.

It should also be noted that in the medical device for rigid endoscopy of the present invention, the connection pins are placed such that if at some point during the intervention the anti-fogging effect of the endoscope had to stop being used, it could continue to be conventionally used with the aim of first enabling the surgical field to be illuminated. That is why the connection pins are located in the ID ring, next to the light post responsible for transmitting the light.

The generality of the light post facilitates the adaptability of existing light cables, enabling the alternative use to the anti-fogging system.

Moreover, it should be noted that the location of the connection pins in that part of the endoscope (ID ring 4) facilitates the adaptability.

Thus, the medical device for rigid endoscopy of the present invention is a novel system in terms of the product design focused on the optimisation and an easy manufacturing without risks for the user and for the product itself.

### EXPLANATION OF THE INVENTION

The present invention is an anti-fogging system that can be thermoregulated by means of a micro controller, this system is implemented in the manufacturing process of any rigid endoscope. For which reason, the temperature thereof is controlled so that in no case is the endoscope fogged during the intervention.

This invention may be applied in any type of endoscopic surgery, wherein an optic is introduced into a cavity of an organism, human or animal. This makes endoscopic surgery the field of application thereof. This overcomes the difficulties of loss of vision in the surgical field caused by fogging, which have not been solved until now.

The fogging of the tip of the optic or distal end is caused by the difference in temperature between the two faces of the glass of the tip. Air has a capacity to retain humidity in the form of vapour which has a direct relationship with the temperature. Thus, when a volume of air which retains a certain volume of water vapour cools, the retention capacity thereof will decrease and the excess humidity will condense. The film of air which is in contact with a cold surface is forced to shed the excess humidity and it is this condensation which clouds the glass of the optic.

The following cause the fogging of the lens:
- The low temperature of the insufflated gas (CO2), which is used in certain surgeries such as gastric surgery.
- The body temperature itself of the patient.
- The humidity existing inside the surgical field due to the presence of bodily fluids.
- The interaction of the surgical instruments and the endoscope.
- External variables.

Currently in surgical practice, lens fogging is fought in two different moments:

### Before the intervention.

- Immersing the optic in hot serum at 50 °C before introducing it into the cavity, in order to heat the walls thereof and prevent it from being a cold surface, whereon water vapour condenses.
- Increasing the temperature of the CO2 through a heated insufflator duct.

### During the intervention:

- Applying anti-fogging agents on the tip of the optic, such as isopropyl alcohol, and other products present on the market.
- Manual cleaning of the lens by means of gauze pads and immersion of the endoscope in hot serum at 50 °C.
- Introducing the distal tip of the endoscope into auxiliary heating devices.

However, these are only temporary solutions, since the moment the optic loses temperature, the lens fogs up again.

These still unsolved difficulties cause:
- Loss of vision in the surgical or examined field.
- Loss of time during the intervention caused by the continuous repetitions in the extraction and insertion of the endoscope in the body during the intervention in order to defog it and continue with the surgery or medical examination.
- High cost in consumable CO2 probes

The main advantages of this invention are as follows:
- The surgeon will always have a clear vision. This way, they can control the working temperature of the endoscope in a range between 30 and 50 degrees Celsius.
- It prevents the continuous repetitions in the extraction and insertion of the endoscope in the body during the intervention in order to defog it and continue with the surgery or medical examination. This causes a significant reduction in the time of the intervention, since it is not necessary to perform continuous manoeuvres in order to defog it on the outside. Likewise, there is greater control over possible haemorrhages which may arise during the intervention.
- Lower cost of consumables, thus having significant financial savings. In the case of using the heated CO2 insufflator, since the (consumable) probe used is single-use and has a high economic cost.
- The endoscope, once this anti-fogging system has been installed, does not change the size, length or weight of a standard endoscope. Therefore, it is not necessary to change the operating room instruments, such as the trocars.
- External variables which are difficult to control can be corrected, which directly affect the time and result of the operation: The surgeon prevents the fogging which can be caused by the ambient conditions in the operating room. The use of the rest of the surgical instruments which are introduced into the body of the patient simultaneously with the endoscope, such as cauterisers, or any other instrument which can generate heat. All these instruments generate heat inside the cavity wherein the intervention is being performed, which directly affects the viewing time before the lens is fogged. The same occurs with the anthropometric characteristics themselves of the patient.

### DESCRIPTION OF THE INVENTION

The anti-fogging thermocontrol system, object of the invention, consists of installing an electrical resistor made of nichrome (alloy of Nickel and Chromium) insulated with a high-temperature resistant tape in the internal tube. Said electrical resistor is regulatable in temperature, working in a variable range between 30 and 50 degrees Celsius. The thermoregulation is achieved by means of a microcontroller installed in the light source.

The electrical resistor is coupled on the outer surface of the internal tube of the endoscope, radially ending in the distal end. This electrical resistor receives electricity through a connector located in the body of the endoscope; which makes the power sources of the optical fibres of the endoscope and the anti-fogging system independent, even though both are connected to the light source.

### BRIEF DESCRIPTION OF THE DRAWINGS

As a complement to the description provided and for the purpose of helping to make the features of the invention more readily understandable, said description is accompanied by a set of drawings which, by way of illustration and not limitation, represent the following:
Figure 1 shows a side view of the device of the invention without the external tube.
Figure 2 shows a cross section of the side view of the device of the invention assembled in the final product, wherein the exploded view of the different elements making it up can be seen.
Figure 3 shows a side view of the device of the invention assembled in the final product.
Figure 4 shows a detailed view of the connection for the temperature control.

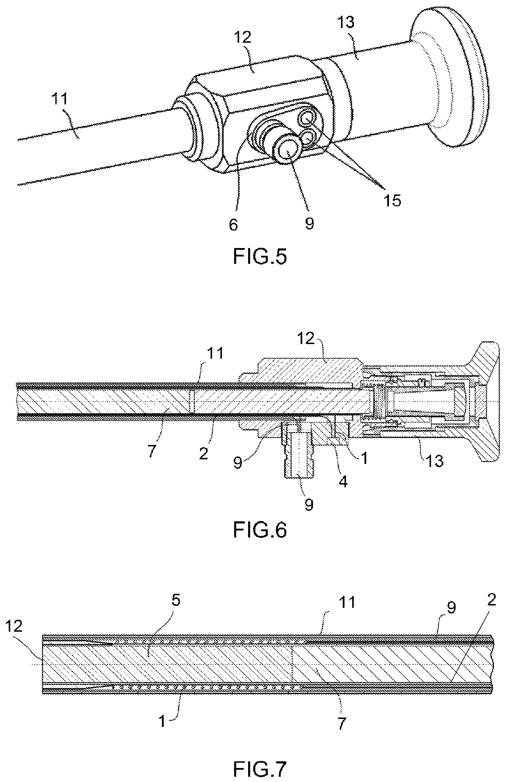

Figure 5 shows another detailed view of the connection for the temperature control.
Figure 6 shows a cross section of what is represented in figure 5.
Figure 7 shows a detailed cross section of a front part of the device of the invention.

### PREFERRED EMBODIMENT OF THE INVENTION

In view of the mentioned drawings and according to the numbering used, the figures show a preferred exemplary embodiment of the invention, which comprises the parts and elements indicated and described in detail below.

The architecture of a rigid endoscope consists mainly of 4 parts:
- External tube (11)
- Body (12)
- Eye piece (13)
- Light post (14)

The external tube (11) houses the internal tube (2) wherein the lenses (7), type 1 spacers (8), type 2 spacers (6) and the objective lens system (5) are located. The optical fibre (9) and the electrical resistor (1) are located between the internal tube (2) and the external tube (11). The electrical resistor (1) is coiled on the surface of the internal tube (2) fully adhered and insulated by a high-temperature resistant tape. Therefore, the optical fibre (9) is located on the electrical resistor (1) already coiled on the internal tube (2), thus occupying the remaining space up to the internal surface of the external tube (11).

The body (12) is the component responsible for assembling each of the subassemblies making up the final product. The eye piece (13) together with the external tube (11) and the light post (14) are gathered therein. Therefore, it is the nexus between the subassemblies responsible for transmitting the vision, transmitting the light, and the thermocontrollable anti-fogging system.

The eye piece (13) therein groups all the elements related to the vision of the proximal end. The focus system (10) is located in this subassembly. The eye piece (13) is connected to the camera in order to be able to project the image obtained by the rigid endoscope on a monitor.

The light post (14) is the assembly responsible for housing the components related to the transmission of light. This assembly is connected directly to the light cable. The optical fibre (9) which extends along the inside of the external tube (11) passes through this assembly.

The electrical resistor (1) which is placed around the internal tube (2) is made of nichrome. Nichrome is an alloy made of nickel and chromium. It stands out for being a metal which is highly resistant to high temperatures and for having a high electrical resistance. The main properties thereof are:
- High resistance to corrosion and oxidation.
- High resistance to high temperatures (high melting point).
- High electrical resistance (it is not as good a conductor as other metals).
- It is not magnetic.
- Silver-grey colour.
- Resistant and flexible.
(https://www.micro-log.com/index.php?controller=attachment&id_attachment=19)

The temperature of the electrical resistor (1) can be regulated by reaching a variable temperature in a thermal interval between 30 and 50 degrees Celsius. The diameter of the electrical resistor (1) is 0.2 mm, the value of the electrical resistor (1) is 180 Ω and the consumption thereof is 1.2 W.

The electrical resistor (1) is placed around the internal tube (2), previously insulated with a polyamide high-temperature resistant tape. Said tape is used successfully in applications at temperatures as low as -269 degrees Celsius and as high as 400 degrees Celsius. The tape has an adhesive film in order to be stuck to the internal tube (2) of the endoscope. It has high performance, reliability and durability, with a unique combination of electrical, thermal, chemical and mechanical properties.
(http://www.dupont.com/content/dam/dupont/products-and-services/membranes-and-films/polyimde-films/documents/DEC-Kapton-HN-datasheet. pdf)

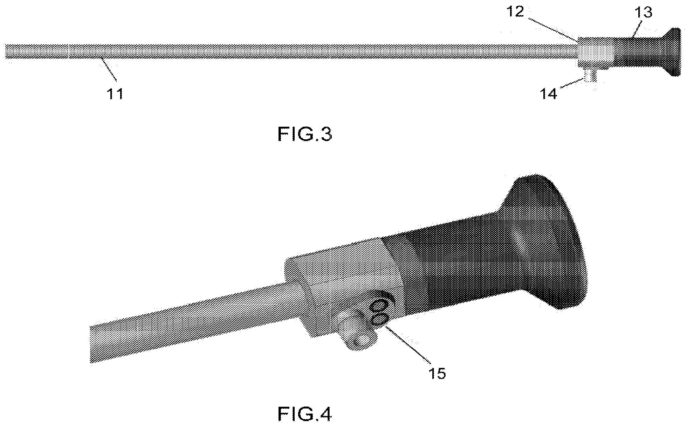

Once the endoscope has been assembled, the electrical resistor (1) will be connected to two connection pins (15) which come out of the body (12) of the endoscope as shown in (FIG. 4) in order to be able to perform the thermal regulation with the equipment to which it will be connected.

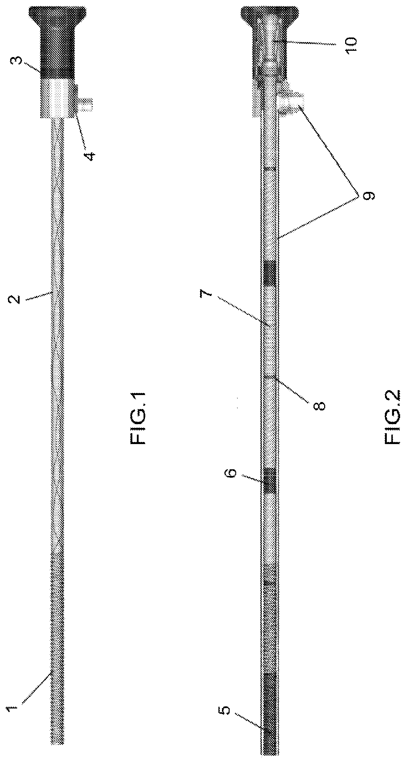

Figure 1 represents the device of the invention with the following elements: the electrical resistor (1), the internal tube (2), an eye piece ring (3) and an ID ring (4).

## Claims

1. A **medical device for rigid endoscopy,** comprising:
- a double coaxial tube structure formed by an external tube (11) and an internal tube (2); wherein between both tubes (2, 11) there is a coaxial space wherein an optical fibre (9) is located along the length of said coaxial space;
- a body (12) including a light post (14) for the optical fibre (9); wherein the body (12) is coupled to a rear end segment of the double coaxial tube structure; and wherein the light post (14) is configured to plug a light cable into it, and this to a light source;
- an eye piece (13) connected to the body (12);
- lenses (7) located inside the internal tube (2);
- an objective lens system (5) located inside a front end section of the internal tube (2); wherein the front end section is located in an area opposite from the rear end section of the double coaxial tube structure;
**characterised in that** it comprises an electrical resistor (1) made of nichrome around the objective lens system (5) which is located inside the coaxial space of the double coaxial tube structure; wherein said electrical resistor (1) is coiled around the internal tube (2) and fully adhered and insulated by means of a high-temperature resistant tape which is stuck to an outer surface of the internal tube (2); and wherein the optical fibre (9) surrounds the electrical resistor (1) which is configured to prevent the fogging of the objective lens system (5).

2. **The medical device for rigid endoscopy,** according to claim 1, **characterised in that** the body (12) includes two connection pins (15) configured for an electrical connection in order to power the electrical resistor (1) for the regulation and control of temperature with electrical current.
